# FASCICULE DE BREVET EUROPEEN

(11) **EP 1 581 833 B1**
(45) Date de publication et mention de la délivrance du brevet: **21.05.2008**
(21) Numéro de dépôt: 03815393.8
(22) Date de dépôt: 12.12.2003
(51) Int. Cl.: G02B 26/08, G02B 21/00, G02B 23/24

(54) **TETE OPTIQUE CONFOCALE MINIATURE A BALAYAGE INTEGRE ET SYSTEME D'IMAGERIE CONFOCALE METTANT EN OEUVRE LADITE TETE**
MINIATURISIERTER KONFOKALER OPTISCHER KOPF MIT INTEGRIERTEM SCAN UND KONFOKALES ABBILDUNGSSYSTEM DAMIT
MINIATURE CONFOCAL OPTICAL HEAD WITH INTEGRATED SCANNING AND CONFOCAL IMAGING SYSTEM USING SAME

(30) Priorité: 20.12.2002 FR 0216279
(43) Date de publication de la demande: 05.10.2005
(73) Titulaire: Mauna Kea Technologies, 75010 Paris (FR)
(72) Inventeur: BERIER, Frédéric, F-92810 PLOUARZEL (FR); VIELLEROBE, Bertrand, F-94130 Nogent sur Marne (FR); GENET, Magalie, F-78280 Guyancourt (FR); LACOMBE, François, F-92370 Chaville (FR); SANTOS, Pedro, f-77140 Saint Pierre Les Nemours (FR)
(74) Mandataire: Pontet, Bernard
(86) Numéro de dépôt international: PCT/FR2003/003686
(87) Numéro de publication internationale: WO 2004/066016

(56) Documents cités:
- WO-A-02/13343
- WO-A-99/00690
- US-A- 6 091 067

## Description

La présente invention concerne une tête optique confocale, notamment miniature, comprenant des moyens de balayage de faisceau intégré et un système d'imagerie confocale équipé de ladite tête.

Plus précisément, la présente invention vise une tête optique et un système optique correspondant, dans lesquels un champ à imager dans un échantillon est balayé point à point par un signal d'excitation. Ce type de système permet d'obtenir, grâce à un traitement informatique, une image confocale construite en temps réel point à point. Le caractère confocal est obtenu grâce à un filtrage spatial permettant de détecter un signal de retour provenant uniquement du point excité de l'échantillon empruntant le même chemin optique que le signal d'excitation.

Un des domaines d'application visé est celui de l'imagerie confocale haute résolution, permettant d'observer et d'analyser un tissu biologique in vivo in situ en temps réel, notamment accessible via le canal opérateur d'un endoscope ou intégré à l'endoscope. L'invention peut également s'appliquer aux domaines de la dermatologie ou de la gynécologie nécessitant une miniaturisation moins poussée de la tête optique. Elle peut également encore s'appliquer au domaine de l'électronique par exemple pour le contrôle de semi-conducteurs ou matériaux similaires.

Selon un premier type de système, notamment décrit dans la demande de brevet WO O0/16151 on utilise un guide d'image, constitué d'un faisceau de fibres optiques souples, comportant à son extrémité distale une tête optique de focalisation destinée à venir en contact de l'échantillon à analyser. Les moyens de balayage du faisceau d'excitation sont situés à l'extrémité proximale du guide d'image prévus pour balayer tour à tour les fibres. Le caractère confocal réside ici notamment dans le fait que la même fibre optique du guide est utilisée pour véhiculer le signal d'excitation et le signal de retour émis. Ce type de système présente l'avantage d'une tête optique simplifiée du point de vue mécanique et comportant ainsi essentiellement des moyens optiques de focalisation pouvant être miniaturisés. En revanche, il présente certains inconvénients, liés à l'utilisation d'une matrice de fibres optiques, notamment le problème de l'échantillonnage du tissu (continuité entre les points d'excitation correspondant à l'éclairage d'une fibre), le problème de l'injection des fibres une à une et des réflexions parasites à l'entrée et à la sortie du guide d'image, le traitement sophistiqué informatique de l'image nécessaire pour corriger ensuite le motif des fibres sur l'image, etc.

Selon un autre type de système connu, les moyens de balayage du faisceau sont situés dans la tête optique à l'extrémité distale d'un guide d'image comprenant une unique fibre optique souple. Le caractère confocal est ici obtenu grâce au fait que la fibre optique est utilisée pour véhiculer le signal d'excitation et de retour émis avec un diamètre de coeur de la fibre approprié.

Les inconvénients de ce type de système sont alors essentiellement liés aux difficultés pour miniaturiser la tête, à la reproductibilité et la fiabilité des moyens mécaniques utilisés pour réaliser le balayage du faisceau émergent de la fibre optique.

Le document US 6,091,067 décrit un système de balayage dans lequel une fibre optique est fixée à deux cales piézoélectriques bimorphes, l'une des cales est placée dans la direction axiale de la fibre et l'autre dans la direction perpendiculaire à l'axe optique. Les cales, pour offrir un débattement approprié par rapport au champ d'observation, doivent présenter une longueur donnée. Perpendiculairement à l'axe de la fibre optique, cette contrainte de longueur conduit en fait à un diamètre de tête optique trop important pour les applications in vivo in situ visées selon la présente invention.

Plusieurs documents décrivent des têtes optiques miniatures confocales mettant en oeuvre des micro miroirs de type micromécaniques (MEMs).

La demande de brevet US 2002/0018276 décrit un système confocal miniature utilisant une fibre optique. La lumière sortant de la fibre est réfléchie sur la partie métallisée d'une lentille. Cette lumière vient ensuite se réfléchir sur un micro miroir MEMs à deux dimensions entourant la fibre. La lumière est alors envoyée vers l'échantillon via un système optique. La lumière revenant de l'échantillon suit le chemin inverse et repasse par la fibre qui sert de filtrage spatial. Le système est miniature, présentant 2 mm de diamètre et 2,5 mm de longueur.

Les brevets US 6,154,305, US 6,088,145, US 6,007,208, US 5,907,425 et US 5,742,419, décrivent une tête confocale dans laquelle le balayage du champ d'observation est réalisé par deux micro miroirs MEMs pivotés électrostatiquement. La tête proposée est miniaturisable mais en revanche offre un champ d'observation de 60 x 60 µm trop petit par rapport aux applications visées selon l'invention correspondant à un champ au minimum de 100 x 100 µm pour pouvoir observer par exemple plusieurs noyaux de 5 µm de diamètre espacés en général de plusieurs dizaines de *µ*m. Le nombre d'image par seconde de 5 à 8 est par ailleurs encore insuffisant pour une imagerie en temps réel (nécessitant un minimum de 10 à 12 images par secondes en mode le plus lent de 640 lignes). Par ailleurs encore, le champ d'observation se trouve parallèle à l'axe de la fibre optique, ce qui peut conduire à des difficultés pratiques d'utilisation (positionnement correct de la sonde).

Les brevets US 6,172,789 et US 6,057,952 décrivent une tête optique confocale avec un champ d'observation cette fois dans l'axe de la fibre optique et permettant un réglage de la profondeur du plan d'observation. Les moyens de balayage comprennent un miroir mobile et un miroir fixe. Le miroir mobile a une ouverture en son centre, et il est de type MEMs, monté de manière à pouvoir pivoter au moins selon une direction. Les surfaces de réflexion des miroirs mobile et fixe sont situées face à face. La lumière sortant de la fibre optique passe à travers l'ouverture du miroir mobile, puis est réfléchie par le miroir fixe en direction du miroir mobile. On rencontre notamment les inconvénients suivants avec une telle construction de tête : un coût important pour le miroir mobile percé au centre, et également la' vitesse de balayage d'un tel miroir MEMs à deux dimensions qui n'est pas suffisante pour une imagerie en temps réel.

D'une manière générale, comme dans le document US 2002/0018276, le changement de direction du faisceau optique par des réflexions successives sur des micro-miroirs entraîne des défauts optiques compliqués à corriger, notamment de la distorsion ou de la courbure de champ.

La présente invention a pour but de pallier ces inconvénients et proposer une tête optique qui soit haute résolution, confocale (offrant une résolution optique axiale sensiblement de 1 à 2 *µ*m) et qui comporte des moyens de balayage intégrés d'un faisceau d'excitation. Selon d'autres objectifs, la tête optique doit pouvoir être miniaturisée (ne dépassant pas 3 mm de diamètre total), permettre la réalisation d'une image en temps réel (au moins 10 images/s) et couvrir un champ à imager de l'ordre au minimum de 100 x 100 *µ*m et de préférence de 150 × 150 *µ*m.

Elle propose une tête optique confocale miniature pour un système d'imagerie confocale, notamment endoscopique, ladite tête comprenant:
- une source ponctuelle produisant un faisceau d'excitation ;
- des moyens optiques adaptés notamment à faire converger ledit faisceau optique en un point d'excitation situé dans un plan subsurfacique par rapport à la surface d'un échantillon, ledit plan étant perpendiculaire à l'axe optique de la tête optique ; et
- des moyens de balayage dudit point d'excitation de manière à décrire un champ d'observation dans ledit plan subsurfacique selon deux directions de balayage perpendiculaires, un balayage rapide en ligne et un balayage lent en colonne,
caractérisée par des moyens micro systèmes mécaniques MEMs adaptés à déplacer en translation selon un débattement choisi au moins l'un des moyens optiques qui est mobile selon une direction perpendiculaire audit axe optique de manière à obtenir au moins une desdites directions de balayage.

Selon l'invention, un balayage latéral de faisceau est réalisé en déplaçant latéralement par rapport à l'axe optique l'une des optiques de transmission, l'axe optique de la tête optique n'est ainsi modifié que latéralement et non angulairement comme avec des optiques de réflexion. Cela a pour avantages de conserver un éclairage axial de l'échantillon et de minimiser le diamètre de la tête optique.

Les moyens micro systèmes mécaniques appartiennent à la famille des micro composants, connus sous le nom générique de MEMs, permettant de réaliser des balayages, des switches, des déflexions (notamment de miroirs) etc., actionnés sous l'impulsion généralement d'un champ électrique.

Les moyens MEMs pouvant être utilisés selon l'invention sont notamment les suivants :
- les actionneurs électrostatiques utilisant la force d'attraction entre deux charges ;
- les actionneurs thermiques utilisant un gradient de température ou la différence de coefficient de dilatation thermique entre deux matériaux, un courant électrique créant par exemple l'élévation de température ;
- les actionneurs magnétiques utilisant la force résultante de l'interaction de deux champs magnétiques ; généralement, un champ externe et un champ interne au MEMs sont appliqués, par exemple une bobine créant un champ externe lorsqu'un courant électrique la traverse.

Les MEMs présentent un temps de réponse compatible avec le déplacement d'une optique, notamment une lentille, sur une distance correspondant, au grandissement près, à la dimension du champ à imager visée selon l'invention, à savoir de l'ordre de 150 µm, et à une vitesse permettant au choix un balayage lent compris sensiblement entre 10 et 15 Hz, correspondant au balayage en colonne du champ à imager, ou rapide sensiblement de 4 kHz, correspondant au balayage en ligne du champ. Les MEMs ont par ailleurs des dimensions compatibles avec une miniaturisation de la tête optique, de l'ordre de 2 à 3 mm de diamètre total, comme le montre la description détaillée donnée plus loin. Ils permettent d'imprimer un mouvement stable et répétable des optiques. De préférence, dans ce sens, les moyens MEMs utilisés selon l'invention sont constitués de moyens de déplacement, par exemple des paires de peignes électrostatiques, disposés de manière diamétralement opposée et actionnés selon différents modes, simultanément ou en alternance. Il existe également d'autres systèmes de MEMs en forme d'anneau dans l'ouverture duquel une lentille peut être assujettie et fonctionnant également en alternance pour imprimer un mouvement en translation en va et vient.

La source ponctuelle peut être fibrée, et donc déportée, : l'extrémité d'une fibre optique souple connectée à une source laser est assujettie à la tête optique. La source peut également être constituée d'un composant VCSEL (« Vertical-Cavity Semiconductor Emitting laser » en langue anglaise) qui est une source laser quasiment ponctuelle ayant une cavité dont la sortie mesure quelques microns de diamètre. Dans les deux cas, la dimension de la source (diamètre de coeur de la fibre optique ou diamètre de sortie de la cavité du composant VCSEL) est choisie pour pouvoir servir de trou de filtrage du signal rétro-émis et réaliser une image confocale. Si l'on-utilise une source VCSEL, un détecteur est placé juste derrière la cavité pour construire la voie de détection. Par ailleurs, l'ouverture numérique de la source, fibrée ou VCSEL, est choisie pour collecter le maximum de photons émis par chaque spot d'excitation, typiquement comprise entre 0,2 et 0,4.

Les moyens optiques utilisés dans la tête optique comprennent généralement des premiers moyens optiques, placés à une distance focale choisie de la source ponctuelle, adaptés à transformer le faisceau d'excitation sortant de la source, qui est divergent du fait de l'ouverture numérique de la source, en un faisceau parallèle ou légèrement divergent. Ces moyens optiques sont constitués d'une lentille ou d'un jeu de lentilles réfractives (standards ou à gradient d'indice) et/ou diffractives. Les lentilles diffractives présentent l'avantage d'être plus petites et plus légères. Des deuxièmes moyens optiques placés à une distance focale choisie des premiers moyens optiques ont pour fonction ensuite de focaliser le faisceau optique en un point d'excitation situé dans le plan subsurfacique d'observation. Ces moyens optiques peuvent également être constitués d'une lentille ou d'un jeu de lentilles réfractives et/ou diffractives.

De préférence, des moyens MEMs sont mis en oeuvre selon l'invention pour déplacer les premiers moyens optiques plutôt que les seconds moyens optiques et à une fréquence permettant de réaliser le balayage en colonne lent. Le balayage des seconds moyens optiques entraînerait des aberrations optiques supplémentaires du fait de leur grande ouverture numérique sur l'échantillon.

Pour ce qui est du balayage rapide en ligne, selon l'invention, celui-ci peut être réalisé soit en déplaçant la source, à l'aide par exemple de moyens piézo-électriques adaptés, soit avec une source fixe et un déplacement des deuxièmes moyens optiques également à l'aide de moyens MEMs appropriés et selon une direction perpendiculaire au déplacement des premiers moyens optiques.

Un balayage supplémentaire en z, pour modifier la profondeur de visualisation, peut en outre être prévu dans la tête optique selon l'invention, par exemple à l'aide de moyens MEMs adaptés à déplacer l'un des moyens optiques mobiles selon l'axe optique de la tête optique, ou à l'aide de moyens adaptés à modifier le rayon de courbure de l'un des moyens optiques, de préférence celui le plus proche de l'échantillon. Un tel balayage permet avantageusement de recréer par traitement informatique une image en 3D.

La présente invention propose également un système d'imagerie confocale mettant en oeuvre une tête optique telle que définie ci-dessus. Selon un premier exemple, le système comprend une source fibrée. Selon un second exemple, il comprend une source de type VCSEL associée à des moyens de détection intégrés dans la tête optique et des moyens de liaison souple entre ladite tête et les moyens de traitement informatique de signal.

La présente invention sera mieux comprise et d'autres avantages apparaîtront plus clairement à la lumière de la description qui va suivre d'exemples de réalisation, description faite en référence aux dessins annexés sur lesquels :
- la figure 1 est un schéma général d'un exemple de système d'imagerie confocale fibrée mettant en oeuvre la tête miniature selon l'invention ;
- la figure 2 est une vue en coupe de côté d'une tête miniature selon un premier exemple de réalisation ;
- la figure 3 est un schéma optique de la tête miniature de la figure 2; et
- la figure 4 est une vue similaire à la figure 2 illustrant un deuxième mode de réalisation possible.

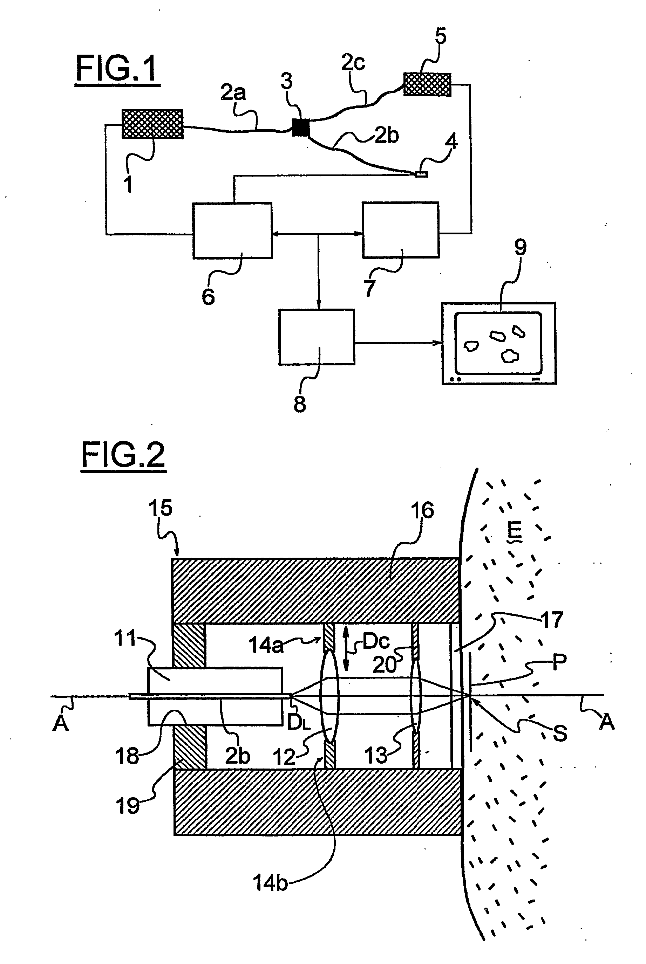

La figure 1 représente schématiquement un système d'imagerie confocale fibrée pouvant inclure une tête miniature selon l'invention.

Le système comprend une source 1, par exemple une source laser, adaptée à émettre un signal d'excitation à une longueur d'onde capable de générer dans un échantillon un signal de retour de fluorescence ou de rétrodiffusion, ledit signal étant transporté par une première fibre optique monomode 2a jusqu'à des moyens de couplage 3, par exemple un coupleur 50/50 de fibre, prévus pour diriger le signal d'excitation provenant de la source 1 dans une fibre optique monomode 2b au bout de laquelle se trouve la tête optique miniature 4 selon l'invention et pour diriger le signal de retour provenant du site excité vers des moyens de détection 5, par exemple un photodétecteur, à l'aide d'une troisième fibre optique monomode 2c. Le système comprend des moyens 6 électroniques de contrôle, de commande et de synchronisation, permettant de piloter la source 1, les moyens de balayage de la tête optique 4 et les moyens de détection 5, de manière synchronisée, afin notamment de connaître l'emplacement du signal dans l'échantillon pour permettre la construction d'une image en temps réel. Le système comprend en outre des moyens 7 électroniques d'amplification, de mise en forme et de conversion A/N du signal détecté par les moyens de détection 5, des moyens 8 informatiques comprenant une carte d'acquisition et une carte graphique et des moyens d'affichage 9 des images obtenues.

Ce système fonctionne globalement de la manière suivante : la tête optique miniature 4 est mise au contact d'un échantillon à analyser, par exemple via le canal opérateur d'un endoscope. La source 1 envoie un signal d'excitation à une longueur d'onde choisie dans la portion de fibre 2a. Le coupleur 3 dirige le signal d'excitation dans la portion de fibre 2b conduisant le signal dans la tête optique 4 où il est balayé et focalisé sur une surface d'analyse (ou champ d'analyse) dans un plan de l'échantillon perpendiculaire à l'axe optique défini par la fibre optique 2b, à une profondeur donnée dans l'échantillon. Un signal de retour issu de la surface balayée dans l'échantillon suit le chemin inverse du signal d'excitation jusqu'au coupleur 3 : il est capté par les moyens optiques de la tête optique 4, recouplé dans la portion de fibre 2b, puis dirigé par le coupleur 3 dans la portion de fibre 2c vers le détecteur 5. Le signal détecté est amplifié et converti en un signal numérique, puis traité de manière informatique pour constituer pour finir un élément d'image affichée en temps réel.

La tête miniature selon l'invention est maintenant décrite en détails en référence à l'exemple de réalisation choisi et représenté à la figure 2.

La tête comprend, dans une structure de support mécanique 15, :
- des moyens de type piézoélectriques 11 auxquels est assujettie la portion terminale de la fibre optique 2b, adaptés à déplacer cette partie terminale de fibre optique, et donc le faisceau optique sortant de celle-ci, sensiblement en translation en va et vient selon un débattement choisi D_{L}, sensiblement perpendiculaire à l'axe optique A défini par la fibre optique, et correspondant à un débattement sur une ligne de champ à imager (ces moyens piézoélectriques sont décrits plus loin en détails) ;
- des premiers moyens optiques 12 adaptés à convertir le faisceau sortant de la fibre 2b qui est divergent en un faisceau parallèle ou légèrement divergent, et à capter le faisceau de retour issu de l'échantillon ; ces moyens optiques sont selon l'invention mobiles en translation, de manière à déplacer le faisceau optique selon un débattement choisi, schématisé selon la double flèche D_{C}, perpendiculairement au débattement D_{L}, et correspondant à un débattement sur une colonne de champ à imager ;
- des moyens micro-mécaniques de type MEMs, 14a et 14b, adaptés à déplacer en va et vient les moyens optiques 12 selon le débattement D_{L} (ces moyens sont décrits plus loin en détails) ;
- des second moyens optiques fixes 13 adaptés à faire converger ledit faisceau parallèle pour le focaliser en un point ou spot S dans un plan P de l'échantillon E examiné situé à une profondeur donnée et sensiblement perpendiculaire à l'axe optique A.

Les moyens piézoélectriques 11 et les moyens MEMs 14a et 14b permettent ainsi conjointement de réaliser le balayage du faisceau focalisé sur le champ à imager, les moyens piézoélectriques 11 servant à réaliser un balayage rapide en ligne et les moyens MEMs 14a et 14b servant à réaliser un balayage lent en colonne.

La structure de support mécanique 15 est constituée d'un corps creux 16, par exemple un porte-optiques tubulaire, avec à une extrémité un hublot d'étanchéité 17, destiné à venir au contact de l'échantillon E, adapté à être traversé par les signaux d'excitation et en retour de rétro-diffusion ou de fluorescence, et avec à son autre extrémité un passage 18 pour la portion terminale de la fibre optique 2b et également pour les moyens piézoélectriques 11. Une paroi de côté 19 entoure le passage 18 pour fermer la tête optique de manière étanche. Les moyens optiques fixes 13 sont fixés et centrés par rapport à l'axe optique A à l'intérieur du corps 16, et les moyens optiques mobiles 12 sont reliés à la face intérieure du corps 16 via les moyens MEMs 14a et 14b comme cela est expliqué plus loin en détails.

La fibre optique 2b couplée à la tête optique est de préférence monomode, permettant une illumination de l'échantillon la plus homogène possible. L'ouverture numérique de la fibre est choisie pour être la plus grande possible pour permettre une collecte optimisée de photons, et pour permettre, conjointement avec un diamètre de coeur approprié, un couplage du signal de retour dans la fibre, et donc un filtrage spatial, qui soit le meilleur possible. Typiquement, l'ouverture numérique est de 0,4 et le diamètre de coeur compris entre 1 et 2 *µ*m. Ce choix de caractéristiques pour la fibre optique, conjointement avec les caractéristiques des moyens optiques 12 et 13, permettent de réaliser un filtrage spatial du signal de retour assurant la confocalité du système.

Selon l'exemple choisi, les moyens piézo-électriques 11 sont constitués d'une cale piézo-électrique présentant une surface sur laquelle est assujettie, par exemple par collage, la partie terminale de la fibre optique 2b avec une portion d'extrémité de la fibre dépassante. Cette cale 11 est double, appelée aussi bimorphe, ayant la particularité de se déformer lorsqu'on lui applique une tension électrique cela permet de déplacer la fibre optique entre une position non excitée ou de repos de la cale et une position excitée correspondant à l'excitation de la cale. La cale 11 est choisie de manière à présenter un débattement entre ces deux positions correspondant, au grandissement près, à la largeur du champ que l'on veut imager dans l'échantillon. En outre, la cale 11 est choisie de manière à pouvoir être excitée à une fréquence permettant de réaliser le balayage rapide souhaité en ligne de l'échantillon. En outre, pour une cale de longueur donnée, compatible au maximum avec une miniaturisation, une amplitude de balayage maximum, peut être obtenue en excitant la cale à sa fréquence de résonance. Typiquement, une cale de 7,5 mm de longueur, excitée avec une tension sinusoidale d'excitation de ±60V, permet d'atteindre une fréquence de résonance de 4kHz, permettant de réaliser un balayage ligne à une vitesse appropriée à l'imagerie en temps réel (au moins 10 images / s), sur un champ imagé de 150 *µ*m de largeur (avec un grandissement unitaire). Cette longueur est compatible avec la dimension totale maximale pour la tête de sensiblement 30 mm. La largeur de la cale (dimension perpendiculairement à la fibre optique) n'influe pas sur la résonance, ni sur l'amplitude de balayage. Typiquement, la largeur de la cale peut être de quelques centaines de microns, ne dépassant pas le diamètre total maximal extérieur prévu pour la tête, sensiblement de 2 à 3 mm. Pour tenir compte d'un grandissement supérieur à 1, pour une dimension de champ imagé donné, on peut choisir une cale piézo-électrique de plus grande longueur pour avoir un débattement plus grand et possédant une plus grande tension d'excitation (±100V à ±120V). Le système comprend en outre les moyens de sécurité nécessaires à l'utilisation de tensions électriques de cet ordre pour des applications in vivo.

Les moyens MEMs 14a et 14b servent à déplacer latéralement les moyens optiques mobiles 12 dans une direction perpendiculaire à celle du mouvement de la fibre imparti par les moyens piézo-électriques. Plus particulièrement selon l'exemple choisi et représenté sur les figures, les moyens optiques mobiles 12 étant constitués d'une lentille simple, les moyens MEMs comprennent deux paires de peignes 14a et 14b, diamétralement opposées, chaque paire de peigne comprend un peigne assujetti à ladite lentille 12 et un peigne assemblé avec le corps 16. Les peignes sont des filaments de semi-conducteur qui sont alignés et les deux peignes d'une paire ont leurs filaments qui se chevauchent. Le mouvement de la lentille 12 est obtenu en décalant un peigne par rapport à l'autre grâce à un effet électrostatique. Le mouvement transversal réalisé grâce aux paires de peignes est perpendiculaire à l'axe de la fibre. Il est réalisé à une fréquence lente (typiquement de 10 à 15 Hz) et permet de réaliser une taille de champ imagé de 150*µ*m en choisissant l'amplitude de balayage appropriée.

L'optique mobile 12 a pour fonction de transformer le faisceau divergent émergent de la fibre optique 2b en un faisceau parallèle ou divergent. Elle doit répondre à des contraintes de dimensions : son diamètre doit être compatible avec une miniaturisation de la tête qui doit présenter un diamètre total extérieur de 2 à 3 mm maximum. Elle doit répondre à des contraintes mécaniques et être suffisamment solide et résistante pour pouvoir être solidaire des moyens MEMs 14 et actionnée en translation. Elle doit répondre également à des contraintes optiques : son diamètre doit être suffisamment grand par rapport au diamètre du faisceau projeté pour le champ imagé que l'on veut obtenir (on risque sinon d'observer des défauts en bord de champ (notamment du vignettage)) ; l'ouverture numérique de l'optique 12 doit être au moins égale ou de préférence supérieure à l'ouverture numérique de la fibre pour conserver la confocalité de la tête et une optimisation du nombre de photons détecté au cours du balayage. Un diamètre minimum d'optique de 1 mm peut être obtenu avec une ouverture numérique supérieure à 0,4 (ON de la fibre optique 2b), ce qui est compatible avec une tête miniature de 2 à 3 mm de diamètre extérieur et l'intercalage des moyens MEMs 14. A titre d'exemple, selon un mode le plus simplifié, cette optique 12 est une lentille classique mais pourrait aussi être un doublet de lentilles ou tout autre optique usuelle permettant de conserver une taille et un poids compatibles avec les objectifs recherchés. A titre d'exemple d'optiques plus sophistiquées, on peut utiliser une lentille diffractive utilisant la diffraction pour décomposer le front d'onde du faisceau incident sur différentes zones et qui après recombinaison permet d'obtenir le front d'onde recherché. En outre, ces optiques présentent les avantages d'une grande ouverture numérique, un faible encombrement, un faible poids et un faible coût.

Les moyens optiques fixes 13 doivent permettre de focaliser le faisceau balayé en un spot S à une certaine profondeur dans l'échantillon dans un plan de focalisation et de collecter un maximum de photons provenant de chaque spot dans ce même plan de focalisation. Les caractéristiques de ces moyens optiques (notamment le grandissement et l'ouverture numérique) sont définies en tenant compte des spécifications de la fibre optique et des moyens optiques mobiles 12 afin d'assurer la confocalité à l'ensemble du système et également pour minimiser les aberrations optiques qui pourraient être engendrées par le balayage (distorsion, instabilité, décalage des moyens optiques mobiles par rapport à l'axe optique, etc.).

Le hublot de sortie 17 est la dernière interface située entre la tête optique et l'échantillon. Il a pour fonction de protéger les optiques, et également de faire une adaptation d'indice entre la tête et l'échantillon, notamment grâce à un traitement de sa face externe par exemple pour un indice de 1,33 égal à celui de l'eau. Le hublot peut également en variante être constitué d'une lentille ayant une fonction de vergence sur le faisceau optique.

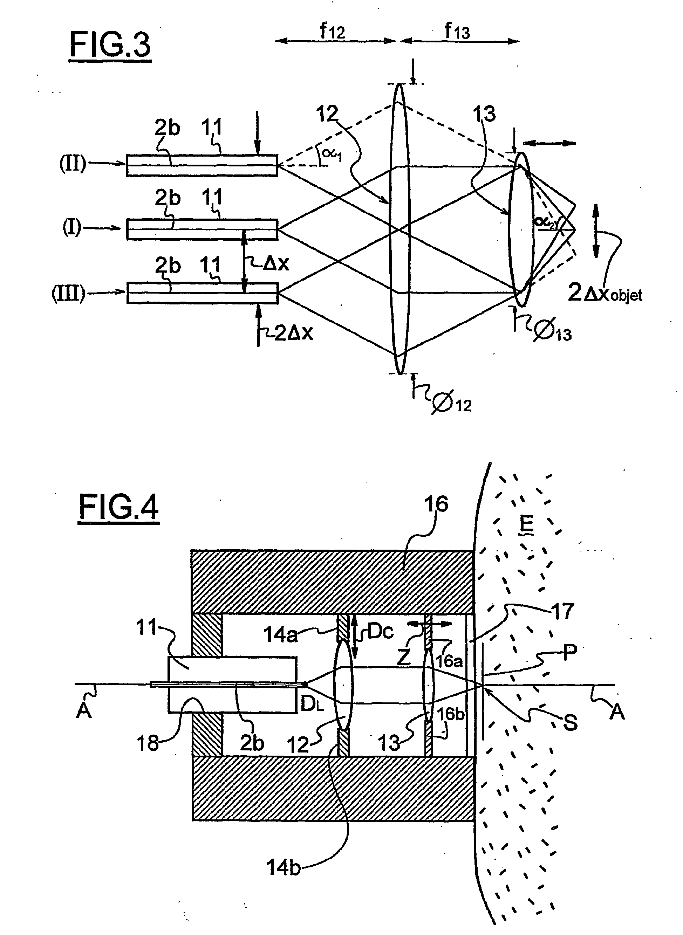

Sur la figure 3 est représenté un schéma optique de la tête qui vient d'être décrite selon un exemple particulier possible, illustrant notamment le balayage en ligne de l'échantillon, sur lequel la fibre 2b est représentée classiquement dans sa position de repos I et dans deux positions latérales extrêmes II et III.

Les spécifications optiques sont les suivantes :
- champ balayé : 2Δx = 400µm
- diamètre de coeur de la fibre 2b : Ø_{coeur} = 1,3 µm
- ouverture numérique de la fibre 2b : ON = sin (α₁) = 0,4
- lentille 12 : diffractive
- focale de la lentille mobile 12 : *f*₁₂ = 1,83mm
- diamètre total de la lentille 12 : Ø₁₂ = 2mm
- lentille 13 : diffractive
- focale de la lentille fixe 13 : *f*₁₃ = 0,73mm
- diamètre total de lentille 13 : Ø₁₃ = 1,6mm,
- grandissement du système optique = G = 2,5
- champ imagé = 2Δx objet = 160 µm
- ouverture numérique objet = ON objet = n sin (α₂) = 1 (dans l'eau n = 1,33)
- diamètre de chaque spot S focalisé dans l'échantillon : limité par la diffraction sur tout le champ imagé.

Dans l'exemple qui vient d'être décrit, les moyens de balayage rapide en ligne du spot d'excitation sont réalisés à l'aide d'une cale piézoélectrique sur laquelle est fixée l'extrémité de la fibre optique. Selon une variante de réalisation possible, la fibre optique est fixe et la lentille 13 est mobile, des moyens MEMs, similaires aux moyens 14a et 14b, étant utilisés pour déplacer la lentille optique 13 selon une direction perpendiculaire au mouvement de translation de l'optique mobile 12 et selon une fréquence compatible avec un balayage rapide en ligne (4Khz). En variante encore, inversement le balayage rapide peut être réalisé en déplaçant la lentille 12 et le balayage lent la lentille 13.

Sur la figure 4, est représentée une variante encore de réalisation de la tête optique de la figure 2, les moyens identiques portant les memes références qu'à la figure 2. Dans cette variante, des moyens 16a et 16b, fixés d'un coté au tube porte optiques 15 et de l'autre à la lentille 13, sont utilisés pour déplacer ladite lentille 13 selon l'axe optique A de la tête, permettant de régler la profondeur de visualisation dans l'échantillon, sensiblement sur quelques dizaines de µm, schématisé par la double flèche Z. Cela permet avantageusement de réaliser des reconstructions en trois dimensions de l'échantillon que l'on observe : on fait différentes acquisitions de données en deux dimensions à différentes profondeurs et l'on peut ensuite reconstruire le volume observé par traitement de données. Dans d'autres cas, le réglage de la profondeur de visualisation peut permettre de s'adapter avantageusement à l'échantillon observé, à la profondeur optimale de visualisation de l'échantillon. Le déplacement de l'optique 13 dans la direction axiale de la tête permet de faire converger le faisceau à une distance différente. Les moyens 16a, 16b sont de type Mems ou piézo-électriques

En variante de réalisation encore, la tête optique peut comprendre à la place de la fibre optique 2b une source ponctuelle de type VCSEL associée à un détecteur placé juste derrière la cavité du VCSEL. A titre d'exemple, en référence à la figure 3, les spécifications optiques suivantes peuvent être données :
- champ balayé : 2Δx = 400- 600*µ*m
- diamètre de l'ouverture de la cavité VCSEL : Ø_{Cavité} = 2-4 *µ*m
- ouverture numérique de la cavité VCSEL : ON = sin (α₁) = 0,25 (dans l'air)
- lentille 12 : diffractive
- focale de la lentille mobile 12 : *f*₁₂ = 3mm
- diamètre total de la lentille 12 : Ø₁₂ = 2mm
- lentille 13 : diffractive
- focale de la lentille fixe 13 : *f*₁₃ = 1,17mm
- diamètre total de lentille 13 : Ø₁₃ = 1,6mm
- grandissement du système optique : G = 3
- champ imagé = 2Δ× objet = 160-240 µm
- ouverture numérique objet = ON objet = n sin (α₂) = 0.75 (dans l'eau n = 1,33)
- diamètre de chaque spot S focalisé dans l'échantillon : limité par la diffraction sur tout le champ imagé.

## Revendications

1. Tête optique confocale miniature (4) pour un système d'imagerie confocale, notamment endoscopique, ladite tête comprenant :
- une source ponctuelle (2b) produisant un faisceau d'excitation ;
- des moyens optiques (12,13) adaptés notamment à faire converger ledit faisceau optique en un point d'excitation (S) situé dans un plan subsurfacique (P) par rapport à la surface d'un échantillon, ledit plan étant perpendiculaire à l'axe optique (A) de la tête optique ; et
- des moyens de balayage dudit point d'excitation de manière à décrire un champ d'observation dans ledit plan subsurfacique selon deux directions de balayage perpendiculaires, un balayage rapide en ligne et un balayage lent en colonne, et
- des moyens micro systèmes mécaniques MEMs (14a-b),
**caractérisée en ce que** ces moyens MEMs sont adaptés à déplacer en translation selon un débattement choisi (Dc) au moins l'un des moyens optiques (12,13) qui est mobile selon une direction perpendiculaire audit axe optique (A) de manière à obtenir au moins une desdites directions de balayage, et **en ce que** ces moyens MEMs sont adaptés à coopérer avec le moyen optique mobile (12,13) de manière diamétralement opposée et en alternance.

2. Tête optique selon la revendication 1, **caractérisée en ce que** le balayage lent correspond à une fréquence sensiblement de 10 à 15 Hz et le balayage rapide à une fréquence sensiblement de 4 kHz, de manière à réaliser une image en temps réel.

3. Tête optique selon l'une des revendications 1 et 2, **caractérisée en ce que** le faisceau optique produit par la source (2b) est divergent, les moyens optiques (12,13) comprenant successivement des premiers moyens (12) adaptés à transformer ledit faisceau divergent en un faisceau parallèle ou légèrement divergent et des seconds moyens optiques (13) adaptés à former le point de focalisation subsurfacique (S).

4. Tête optique selon la revendication 3, **caractérisée en ce que** le premier moyen optique (12) est mobile, adapté à réaliser le balayage lent en colonne du faisceau optique,

5. Tête optique selon l'une des revendications 1 à 4, **caractérisée en ce que** deux des moyens optiques (12,13) sont mobiles, adaptés à être déplacés chacun selon une direction perpendiculaire à l'axe optique de manière à définir chacun une direction de balayage.

6. Tête optique selon l'une des revendications 1 à 4, **caractérisée en ce que** la source (2b) est mobile, assujettie à des moyens de type piezoélectriques (11) adaptés à déplacer le faisceau d'excitation émis par ladite source avec un débattement choisi (D_{L}) de manière à définir la deuxième direction de balayage.

7. Tête optique selon la revendication 6, **caractérisée en ce que** la deuxième direction de balayage et les caractéristiques des moyens piézoélectriques (11) correspondent à un balayage rapide en ligne.

8. Tête optique selon la revendication 7, **caractérisée en ce que** les moyens piézo-électriques comprennent une cale piézoélectrique bimorphe (11) s'étendant en longueur selon l'axe optique (A) de la tête, ladite source (2b) étant fixée sur l'une des faces de ladite cale à l'extrémité avant de la cale en regard des moyens optiques (12,13).

9. Tête optique selon l'une quelconque des revendications précédentes,
**caractérisée en ce qu'**elle comprend des moyens pour modifier la profondeur du plan subsurfacique d'observation (P) dans l'échantillon.

10. Tête optique selon la revendication 9, **caractérisée en ce que** les moyens pour modifier la profondeur du plan subsurfacique d'observation (P) dans l'échantillon comprennent des moyens micro-mécaniques MEMs (16a-b) adaptés à déplacer certains des moyens optiques (13) selon l'axe optique de la tête optique.

11. Tête optique selon la revendication 10, **caractérisée en ce que**, les moyens MEMs (16a-b) sont adaptés à déplacer les deuxièmes moyens optiques de focalisation (13) pour réaliser le déplacement (Z) selon l'axe optique du faisceau optique.

12. Tête optique selon la revendication 9, **caractérisée en ce que** les moyens pour modifier la profondeur du plan subsurfacique d'observation (P) comprennent des moyens pour modifier le rayon de courbure de l'un des moyens optiques.

13. Tête optique selon l'une quelconque des revendications précédentes, **caractérisée en ce qu'**elle comprend la partie terminale d'une fibre optique (2b) adaptée à conduire le signal d'excitation depuis une source extérieure, le faisceau émergent de la fibre constituant la source ponctuelle.

14. Tête optique selon la revendication 13, **caractérisée en ce que** la fibre optique est monomode avec un diamètre de coeur permettant un filtrage spatial du signal de retour et donc assurant la confocalité de la tête, la fibre optique présentant une ouverture numérique la plus grande possible.

15. Tête optique selon l'une quelconque des revendications 1 à 12, **caractérisée en ce que** la source est de type VCSEL, présentant une ouverture numérique et un diamètre de sortie de cavité compatible avec un système confocal, et associée à un détecteur placé derrière la cavité du VCSEL.

16. Tête optique selon l'une quelconque des revendications précédentes, **caractérisée en ce qu'**elle comporte un hublot d'étanchéité (17) à la sortie de la tête optique destiné à venir au contact de l'échantillon et à réaliser une adaptation d'indice.

17. Tête optique selon la revendication 16, **caractérisée en ce que** le hublot a une fonction de vergence sur le faisceau optique focalisé.

18. Tête optique selon l'une des revendications précédentes, **caractérisée en ce que** les moyens optiques (12,13) présentent une ouverture numérique au moins égale à l'ouverture numérique de la source.

19. Système d'imagerie confocale comportant :
- une tête (4) optique confocale de focalisation à balayage de faisceau intégré;
- une source (1,2a,2b) adaptée à émettre un faisceau d'excitation ;
- des moyens de détection (5) d'un signal émis ;
- des moyens électroniques et informatiques de commande et de traitement du signal (6-9) émis adaptés à reconstruire une image confocale d'un champ imagé,
**caractérisé en ce que** la tête optique (4) est selon l'une des revendications précédentes.

20. Système selon la revendication 19, **caractérisé par** une fibre optique (2a) reliée à une source laser (1) et des moyens de couplage (3) pour coupler ladite fibre (2a) avec la fibre optique de transport (2b) jusqu'à et depuis la tête optique (4) et une fibre de transport (2c) du signal émis jusqu'aux moyens de détection.

21. Système selon la revendication 19, **caractérisé en ce que**, la tête optique comprenant une source VCSEL et un détecteur intégré, le système comprend des moyens de liaison souple entre la tête optique et les moyens de traitement de signal.

## Claims

1. Miniature confocal optical head (4) for a confocal imaging system, in particular endoscopic, said head comprising:
- a point source (2b) producing an excitation beam;
- optical means (12, 13) capable, in particular, of causing said optical beam to converge into an excitation point (S) situated in a subsurface plane (P) relative to the surface of a specimen, said plane being perpendicular to the optical axis (A) of the optical head; and
- means of scanning said excitation point so as to describe a field of view in said subsurface plane in two perpendicular scanning directions, rapid line scanning and slow column scanning, and mechanical micro-system means MEMs (14a-b), **characterized in that** said MEMs means is capable of moving in translation along a chosen displacement (Dc) at least one of the optical means (12, 13) which is mobile in a direction perpendicular to said optical axis (A) so as to obtain at least one of said scanning directions, and **in that** said MEMs means is capable of cooperating with the mobile optical means (12, 13) in a diametrically opposite manner and alternately..

2. Optical head according to claim 1, **characterized in that** the slow scanning corresponds to a frequency of approximately 10 to 15 Hz and the rapid scanning to a frequency of approximately 4 kHz, so as to produce an image in real time.

3. Optical head according to one of claims 1 and 2, **characterized in that** the optical beam produced by the source (2b) is divergent, the optical means (12, 13) comprising successively first means (12) capable of transforming said divergent beam to a parallel or slightly divergent beam and second optical means (13) capable of forming the subsurface focusing point (S).

4. Optical head according to claim 3, **characterized in that** the first optical means (12) is mobile, capable of carrying out optical beam slow column scanning.

5. Optical head according to one of claims 1 to 4, **characterized in that** two of the optical means (12, 13) are mobile, each capable of being moved in a direction perpendicular to the optical axis so that each defines a scanning direction.

6. Optical head according to one of claims 1 to 4, **characterized in that** the source (2b) is mobile, fixed to piezoelectric-type means (11) capable of moving the excitation beam emitted by said source with a displacement (D_{L}) chosen so as to define the second scanning direction.

7. Optical head according to claim 6, **characterized in that** the second scanning direction and the characteristics of the piezoelectric means (11) correspond to rapid line scanning.

8. Optical head according to claim 7, **characterized in that** the piezoelectric means comprises a bimorphic piezoelectric positioner (11) extending along according to the optical axis (A) of the head, said source (2b) being fixed on one of the faces of said positioner at the front end of the positioner facing the optical means (12, 13).

9. Optical head according to any one of the preceding claims, **characterized in that** it comprises means for modifying the depth of the subsurface observation plane (P) in the specimen.

10. Optical head according to claim 9, **characterized in that** the means for modifying the depth of the subsurface observation plane (P) in the specimen comprises micro-mechanical means MEMs (16a-b) capable of moving certain optical means (13) along the optical axis of the optical head.

11. Optical head according to claim 10, **characterized in that** the MEM means (16a-b) is capable of moving the second optical focusing means (13) in order to carry out the movement (Z) along the optical axis of the optical beam.

12. Optical head according to claim 9, **characterized in that** the means for modifying the depth of the subsurface observation plane (P) comprises means for modifying the radius of curvature of one of the optical means.

13. Optical head according to any one of the preceding claims, **characterized in that** it comprises the terminal part of an optical fibre (2b) capable of guiding the excitation signal from an external source, the emergent beam from the fibre constituting the point source.

14. Optical head according to claim 13, **characterized in that** the optical fibre is single-mode with a core diameter allowing spatial filtering of the return signal and therefore ensuring the confocality of the head, the optical fibre having as large a numerical aperture as possible.

15. Optical head according to any one of claims 1 to 12, **characterized in that** the source is of VCSEL type, having a numerical aperture and a cavity outlet diameter compatible with a confocal system, and associated with a detector placed behind the cavity of the VCSEL.

16. Optical head according to any one of the preceding claims, **characterized in that** it comprises a tight window (17) at the outlet of the optical head intended to come into contact with the specimen and in order to carry out an index matching.

17. Optical head according to claim 16, **characterized in that** the window has a refractive power function on the focused optical beam.

18. Optical head according to one of the preceding claims, **characterized in that** the optical means (12, 13) has a numerical aperture at least equal to the numerical aperture of the source.

19. Confocal imaging system comprising:
- a focusing confocal optical head (4) with integrated beam scanning;
- a source (1, 2a, 2b) capable of emitting an excitation beam;
- means (5) of detecting an emitted signal;
- electronic and data processing means for controlling and processing the emitted signal (6-9) capable of reconstructing a confocal image of an imaged field,
**characterized in that** the optical head (4) is according to one of the preceding claims.

20. System according to claim 19, **characterized by** an optical fibre (2a) linked to a laser source (1) and coupling means (3) for coupling said fibre (2a) to the optical fibre (2b) for conveying to and from the optical head (4) and a fibre (2c) for conveying the emitted signal to the detection means.

21. System according to claim 19, **characterized in that** the optical head comprising a VCSEL source and an integral detector, the system comprises flexible linking means between the optical head and the signal processing means.

## Patentansprüche

1. Miniaturisierter konvokaler optischer Kopf (4) für ein insbesondere endoskopisches konfokales Bildherstellungssystem, wobei dieser Kopf umfasst:
- eine punktförmige Quelle (2b), die einen Erregungsstrahl erzeugt;
- optische Mittel (12, 13), die insbesondere dafür ausgelegt sind, den optischen Strahl an einem Erregungspunkt (S) konvergieren zu lassen, der in einer unter der Oberfläche einer Probe gelegenen Ebene (P) liegt, wobei diese Ebene zur optischen Achse (A) des optischen Kopfs senkrecht ist; und
- Mittel zum Abtasten des Erregungspunkts, so dass in der unter der Oberfläche gelegenen Ebene ein Beobachtungsfeld gemäß zwei zueinander senkrechten Abtastrichtungen beschrieben wird, und zwar eine schnelle Abtastung in Zeilen und eine langsame Abtastung in Spalten, und
- mechanische Mikrosystemmittel MEMs (14a-b),
**dadurch gekennzeichnet, dass**
die Mittel MEMs dafür ausgelegt sind, mindestens eines der optischen Mittel (12, 13), das in einer zu der optischen Achse (A) senkrechten Richtung beweglich ist, gemäß einem gewählten Ausschlag (Dc) in Translation zu bewegen, so dass mindestens eine der Abtastrichtungen erhalten wird, und dass die Mittel MEMs dafür ausgelegt sind, mit dem beweglichen Mittel (12, 13) auf diametral entgegengesetzte Weise und abwechselnd zusammenzuwirken.

2. Optischer Kopf nach Anspruch 1, **dadurch gekennzeichnet, dass** die langsame Abtastung einer Frequenz von im Wesentlichen 10 bis 15 Hz und die schnelle Abtastung einer Frequenz von im Wesentlichen 4 kHz entspricht, so dass ein Bild in Echtzeit hergestellt wird.

3. Optischer Kopf nach einem der Ansprüche 1 und 2, **dadurch gekennzeichnet, dass** der von der Quelle (2b) erzeugte optische Strahl divergierend ist, wobei die optischen Mittel (12, 13) nacheinander erste Mittel (12), die dafür ausgelegt sind, den divergierenden Strahl in einen parallelen oder leicht divergierenden Strahl zu überführen, und zweite optische Mittel (13) umfassen, die dafür ausgelegt sind, den unter der Oberfläche liegenden Fokussierungspunkt (S) zu bilden.

4. Optischer Kopf nach Anspruch 3, **dadurch gekennzeichnet, dass** das erste optische Mittel (12) beweglich ist und dafür ausgelegt ist, die langsame Abtastung in Spalten des optischen Strahls durchzuführen.

5. Optischer Kopf nach einem der Ansprüche 1 bis 4, **dadurch gekennzeichnet, dass** zwei der optischen Mittel (12, 13) beweglich sind und dafür ausgelegt sind, jeweils gemäß einer zur optischen Achse senkrechten Rlichtung bewegt zu werden, so dass jeweils eine Abtastrichtung definiert wird.

6. Optischer Kopf nach einem der Ansprüche 1 bis 4, **dadurch gekennzeichnet, dass** die Quelle (2b) beweglich ist, wobei sie durch piezoelektrische Mittel (11) gesteuert ist, die dafür ausgelegt sind, den von der Quelle gesendeten Erregungsstrahl mit einem gewählten Ausschläg (D_{L}) so zu bewegen, dass die zweite Abtastrichtung definiert wird.

7. Optischer Kopf nach Anspruch 6, **dadurch gekennzeichnet, dass** die zweite Abtastrichtung und die Merkmale der piezoelektrischen Mittel (11) einer schnellen Zeilenabtastung entsprechen.

8. Optischer Kopf nach Anspruch 7, **dadurch gekennzeichnet, dass** die piezoelektrischen Mittel eine bimorphe piezoelektrische Scheibe (11) umfassen, die sich in der Länge gemäß der optischen Achse (A) des Kopfs erstreckt, wobei die Quelle (2b) auf einer der Seiten der Scheibe am vorderen Ende der Scheibe gegenüber den optischen Mitteln (12, 13) befestigt ist.

9. Optischer Kopf nach einem der vorhergehenden Ansprüche, **dadurch gekennzeichnet, dass** er Mittel zum Ändern der Tiefe der unter der Oberfläche gelegenen Beobachtungsebene (P) in der Probe umfasst.

10. Optischer Kopf nach Anspruch 9, **dadurch gekennzeichnet, dass** die Mittel zum Ändern der Tiefe der unter der Oberfläche gelegenen Beobachtungsebene (P) in der Probe mikromechanische Mittel MEMs (16a-b) umfassen, die dafür ausgelegt sind, manche der optischen Mittel (13) in der optischen Achse des optischen Kopfes zu bewegen.

11. Optischer Kopf nach Anspruch 10, **dadurch gekennzeichnet, dass** die Mittel MEMs (16a-b) dafür ausgelegt sind, die zweiten optischen Fokussierungsmittel (13) zu bewegen, um die Bewegung (Z) des optischen Strahls in der optischen Achse vorzunehmen.

12. Optischer Kopf nach Anspruch 9, **dadurch gekennzeichnet, dass** die Mittel zum Ändern der Tiefe der unter der Oberfläche gelegenen Beobachtungsebene (P) Mittel zum Ändern des Krümmungsradius eines der optischen Mittel umfassen.

13. Optischer Kopf nach einem der vorhergehenden Ansprüche, **dadurch gekennzeichnet, dass** er den Endteil einer optischen Faser (2b) umfasst, die dafür ausgelegt ist, das Erregungssignal von einer äußeren Quelle an zu leiten, wobei der aus der Faser austretenden Strahl die punktförmige Quelle bildet.

14. Optischer Kopf nach Anspruch 13, **dadurch gekennzeichnet, dass** die optische Faser monomodal mit einem Kerndurchmesser ist, der eine räumliche Filterung des zurückkehrenden Signals gestattet und damit die Konfokalität des Kopfes gewährleistet, wobei die optische Faser die größtmögliche numerische Öffnung aufweist.

15. Optischer Kopf nach einem der Ansprüche 1 bis 12, **dadurch gekennzeichnet, dass** die Quelle eine VCSEL-Quelle ist, die eine numerische Öffnung und einen Hohlraumaustrittsdurchmesser aufweist, der mit einem konfokalen System kompatibel ist, und mit einem hinter dem Hohlraum des VCSEL angeordneten Detektor kombiniert ist.

16. Optischer Kopf nach einem der vorhergehenden Ansprüche, **dadurch gekennzeichnet, dass** er am Austritt des optischen Kopfes ein Dichtungsfenster (17) umfasst, das dazu bestimmt ist, mit der Probe in Kontakt zu kommen, um eine Indexanpassung vorzunehmen.

17. Optischer Kopf nach Anspruch 16, **dadurch gekennzeichnet, dass** das Fenster auf den fokussierten optischen Strahl eine lichtbrechende Funktion ausübt.

18. Optischer Kopf nach einem der vorhergehenden Ansprüche, **dadurch gekennzeichnet, dass** die optischen Mittel (12, 13) eine numerische Öffnung aufweisen, die mindestens gleich der numerischen Öffnung der Quelle ist.

19. Konfokales Bildherstellungssystem, umfassend:
- einen konfokalen optischen Fokussierungskopf (4) mit integrierter Strahlabtastung;
- eine Quelle (1, 2a, 2b), die dafür ausgelegt ist, einen Erregungsstrahl zu senden;
- Mittel (5) zur Erfassung eines gesendeten Signals;
- Elektronik- und Informatikmittel zur Steuerung und zur Verarbeitung des gesendeten Signals (6 bis 9), die dafür ausgelegt sind, ein konfokales Bild eines Bildfelds zu rekonstruieren,
**dadurch gekennzeichnet, dass** der optische Kopf (4) gemäß einem der vorhergehenden Ansprüche ist.

20. System nach Anspruch 19, **gekennzeichnet durch** eine mit einer Laserquelle (1) verbundene optische Faser (2a) und Kopplungsmittel (3) zum Koppeln der Faser (2a) mit der optischen Transportfaser (2b) bis zu und von dem optischen Kopf (4) und eine Faser (2c) zum Transport des gesendeten Signals bis zu den Erfassungsmitteln.

21. System nach Anspruch 19, **dadurch gekennzeichnet, dass**, wenn der optische Block eine VCSEL-Quelle und einen integrierten Detektor umfasst, das System Mittel zur flexiblen Verbindung zwischen dem optischen Kopf und den Signalverarbeitungsmitteln umfasst.
